# EUROPEAN PATENT SPECIFICATION

(11) **EP 2 366 499 B1**
(45) Date of publication and mention of the grant of the patent: **19.08.2015**
(21) Application number: 09771750.8
(22) Date of filing: 12.11.2009
(51) Int. Cl.: B25B 23/142

(54) **WRENCH FOR DELIVERING MAXIMUM FIXED OR ADJUSTABLE TORQUE**
SCHLÜSSEL ZUR ABGABE EINES MAXIMALEN FESTEN ODER EINSTELLBAREN DREHMOMENTS
CLÉ POUR EXERCER UN COUPLE MAXIMAL FIXE OU RÉGLABLE

(30) Priority: 14.11.2008 ES 200803254
(43) Date of publication of application: 21.09.2011
(73) Proprietor: Biotechnology Institute, I Mas D, S.L., 01005 Vitoria (Álava) (ES)
(72) Inventor: ANITUA ALDECOA, Eduardo, 01005 Vitoria (ES)
(74) Representative: Urteaga Simarro, José Antonio
(86) International application number: PCT/ES2009/000531
(87) International publication number: WO 2010/055177

(56) References cited:
- EP-A2- 1 112 818
- DE-B- 1 182 910
- DE-C- 675 755
- FR-A1- 2 216 065
- US-A- 2 125 945
- US-A- 2 674 108
- US-A- 4 535 659
- US-A- 5 224 403
- US-A- 5 859 371

## Description

### Technical field

The invention relates to a wrench capable of providing a fixed or adjustable maximum torque to an external part (a screw, a nut, etc.) and cause said part to rotate.

### Prior art

Wrenches designed to provide a fixed or adjustable maximum torque to an external part (a screw, a nut, etc.) are essentially manual tools that are used to tighten or loosen external parts that, because of their mechanical properties or their operating conditions, require a very specific tightening torque or a tightening torque that does not exceed a specific value. Usually, these types of wrenches comprise an elastic member with a preload that varies according to the tightening torque required. Two types of wrenches are used at present: torque-indicating wrenches and torque-limiting wrenches.

Torque-indicating wrenches feature a visual scale that allows the user to select the torque to be applied on the external part. This type of wrench (examples of which are described in US3670602 and US4827813) has an indicator that makes torque selection easier.

Torque-limiting wrenches (examples of which are described in US3701295, GB1436492 and WO2006/029542A1) are wrenches that allow the provision of a specific or fixed torque only. This type of wrench may be adjustable (e.g. GB1436492), in other words the wrench may allow to select the magnitude of the torque.

Another example of a torque-limiting tool whose maximum torque is adjustable is shown in US5224403. Said wrench comprises an inner spring that engages with a gear at the head of the wrench. The spring is in the shape of an elongated flexible part capable of bending transversally and eventually disengaging from the gear.

Known wrenches present certain drawbacks such as the fact that they are difficult to use, cannot be used in a healthcare environment, and that the user has to exert a great deal of effort in order to apply the torque, etc.

In specific terms, coil-spring-based wenches can be difficult to use because the higher the torque value the user wishes to adjust the wrench to, the greater amount of effort the user has to make to adjust the wrench (select the torque value). As a result, high torque values cannot be achieved in spring-based wrenches as this would require exerting an impossible amount of effort. In addition, spring-based wrenches suffer from what is known as "creep" (an increase in the deformation of a material when constant stress is applied to it), which causes the spring to become less tense and alters the torque scale. An example of a torque-limiting wrench where the maximum torque is controlled by a coil-spring mechanism is shown in US5859371.

This invention aims to resolve these drawbacks affecting existing wrenches.

### Brief description of the invention

It is an object of the invention to provide a wrench that allows a maximum torque to be applied to a part (a screw, a nut, etc.) for the purpose of making the part rotate, wherein the torque provided by the wrench is limited by the controlled buckling of certain internal elongated metal parts of the wrench. The inventive wrench thus comprises at least one elongated metal part provided with a fixed end and a pusher end. The pusher end pushes a pusher member that acts on a torque-applying head. The elongated metal part is capable of withstanding buckling when torque is applied with the wrench. As the applied torque increases, the buckling increases, thus increasing the force that the elongated metal part exerts on the torque-applying head. At a given moment, the elongated metal part reaches a maximum specific buckling point, at which point the wrench provides its maximum torque. The maximum torque is thus determined by the maximum buckling of the elongated metal part.

In one embodiment of the invention, the pusher member pushes a catch comprised in the torque-applying head. When the elongated metal part reaches the maximum buckling point, the pusher end exerts a force on the torque-applying head that is high enough so that the torque-applying head jumps to the next position, thus limiting the torque provided by the wrench. The torque-applying head preferably emits a "click" sound when it jumps to the next position, warning the user that the wrench has reached its maximum torque.

In another embodiment of the invention, the elongated metal part is comprised in a set of parts connected by means of an articulated joint to the torque-applying head. In this embodiment the point at which maximum torque has been reached is indicated by the set of parts rotating in relation to the torque-applying head to an angle of maximum rotation, and not by a "click" or other indication emitted by the torque-applying head, as in the preceding embodiment.

Various embodiments are contemplated depending on the buckling length and the momentum of the elongated metal part. Embodiments are envisaged in which the buckling length and the momentum are fixed, therefore leading to fixed maximum torque wrenches (wrenches whose maximum torque is not user-adjustable). Alternatively, embodiments are envisaged in which either the buckling length or the momentum is variable (user adjustable), and which, therefore, provide wrenches with an adjustable maximum torque. Embodiments are also contemplated in which both the buckling length and the momentum are adjustable.

The inventive wrench offers certain advantages over conventional spring-based wrenches. Firstly, it is easier to use as the effort required to adjust the torque is minimal and does not depend on the torque value to which the wrench is adjusted. In conventional wrenches comprising springs, the user must exceed the preload force of the spring; given the fact that the greater the torque applied, the greater the deformation of the spring, the preload force also increases (as established in Hooke's Law), with the user thus being required to make an increasing amount of effort. In the wrench based on buckling, however, there is no preload force that forces the user to make a greater effort. This also leads to an additional advantage, which is that the wrench based on buckling can provide greater torques.

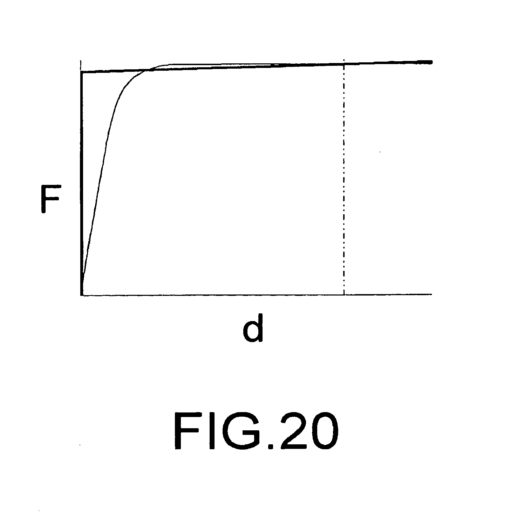

The graph of Figure 20, which shows the force that has to be applied on the spring (the thick stepped line) and on the inventive elongated metal part (the thin curved line) in relation to the required movement or deformation of these parts, provides a better understanding of this phenomenon. As shown, the force the spring has to exert on the pusher end to generate a movement "d" equal to zero is very high, practically the maximum force. In the invention, however, the force on the pusher end in the rest position is zero as the elongated metal part is not bent. This effect ensures that barely any force is required to adjust the inventive wrench. The broken line indicates the maximum point of movement at which the ratchet jumps to the next tooth.

A further advantage of the invention is the fact that the material the elongated metal part is made from only works when torque is applied, thereby preventing "creep" from occurring.

### Brief description of the figures

Details of the invention can be seen in the accompanying non-limiting figures:
- Figure 1 shows a first embodiment of the wrench according to the invention, where the maximum torque that may be provided by the wrench is fixed.
- Figure 2 shows an exploded view of the preceding wrench.
- Figure 3 shows a second embodiment of the wrench according to the invention, where the maximum torque that may be provided by the wrench is variable.
- Figure 4 shows an exploded view of the preceding wrench.
- Figure 5 shows two longitudinal cross-sectional views of the wrench of Figures 3 and 4.
- Figure 6 shows a third embodiment of the wrench according to the invention, where the maximum torque that may be provided by the wrench is variable.
- Figure 7 shows the moving cover of Figure 3.
- Figure 8 shows the moving cover of Figure 6.
- Figure 9 shows an alternative embodiment of the elongated metal part.
- Figures 10 to 13 show the control part of Figure 9 in a variety of positions.
- Figures 14 and 15 show another embodiment of the invention, adjusted in two different ways.
- Figures 16 and 17 respectively show a perspective and a cross-sectional perspective of another embodiment of the invention.
- Figures 18 and 19 show an elevated cross-sectional view of the wrench of Figures 16 and 17 in its initial position and in its position of maximum torque, respectively.
- Figure 20 shows a graph of the force that has to be applied on the spring and on the inventive elongated metal part in relation to the required movement or deformation of these parts.

### Detailed description of the invention

The wrench according to the invention, which allows a maximum torque to be provided to a rotatable external part (e.g. a screw, a nut, etc.), is characterised in that it comprises at least one elongated metal part that pushes a torque-applying head. The elongated metal part is capable of buckling, with the result that when the load reaches a maximum amplitude due to the buckling, the force exerted by the elongated metal part on the torque-applying head is not able to keep the torque-applying head in its position. The torque-applying head then jumps to a next position on a gearwheel, thereby reducing the torque again. As a result, the maximum buckling load of the elongated metal part determines the maximum torque that the wrench is able to provide.

The wrench according to the invention may present a fixed maximum torque or an adjustable maximum torque, depending on whether the buckling length and the momentum of the elongated metal part are fixed or variable.

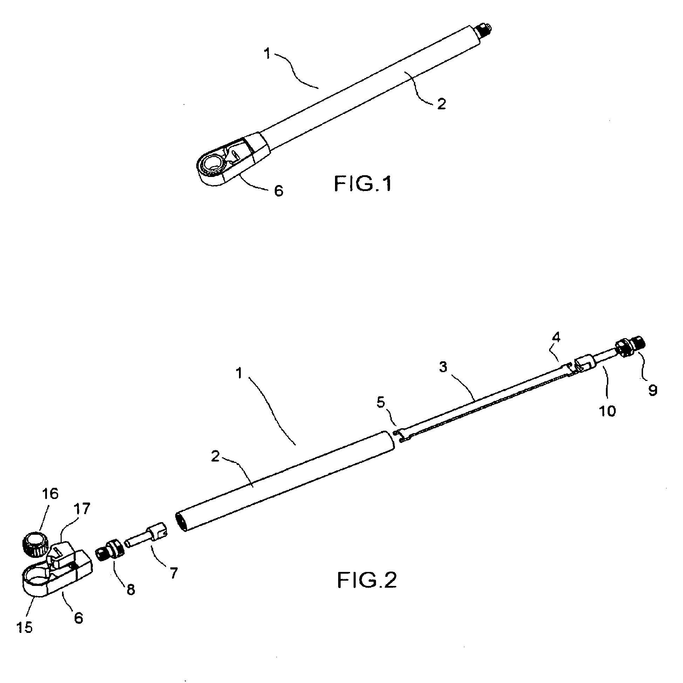

Figure 1 shows a first embodiment of the inventive wrench (1). In said embodiment, the maximum torque that the wrench (1) is able to provide is fixed. The wrench (1) comprises a fixed cover (2) that acts as a handle. A torque-applying head (6) is located on one end of the fixed cover (2). The torque-applying head (6) is a set of parts that enable virtually free rotation in one direction and which control (by means of the force exerted on a gearwheel) the maximum torque that may be applied in the other direction (the direction of tightening).

Figure 2 shows an exploded view of the preceding wrench (1). In addition to the torque-applying head (6) and the fixed cover (2), the wrench (1) comprises an elongated metal part (3) that is housed inside the fixed cover (2). One of the ends of the elongated metal part (3) is a fixed end (4), while the opposite end is a pusher end (5). The pusher end (5) pushes a catch (17) of the torque-applying head (6). The torque-applying head (6) shown in the figure comprises a casing (15) that provides structural support to the torque-applying head (6) and maintains its internal parts isolated from the exterior, a gearwheel (16) that allows the relative rotation of the external part (a screw, etc.) in relation to the wrench (1) and the catch (17) whose function it is to apply the force exerted by the pusher (7) on the gearwheel (16) in an efficient manner.

As shown, the pusher end (5) of the elongated metal part (3) pushes a pusher member (7), which in turn pushes the catch (17). On the other side, the fixed end (4) pushes another pusher member (10). A stopper member (8) connects the torque-applying head (6) to the fixed cover (2) and also limits the axial movement exerted on the pusher (7). Another stopper member (9) limits the movement of the pusher (10). The elongated metal part (3) is capable of buckling when the catch (17) offers resistance, the wrench (1) working as follows: the user turns the wrench (1) with an increasing amount of torque, until the elongated metal part (3) reaches a specific load that causes buckling. Eventually, the buckling makes the pusher end (5) exert sufficient force on the catch (17) so that the catch (17) jumps to the next position of the gearwheel (16), thereby limiting the torque provided by the wrench (1).

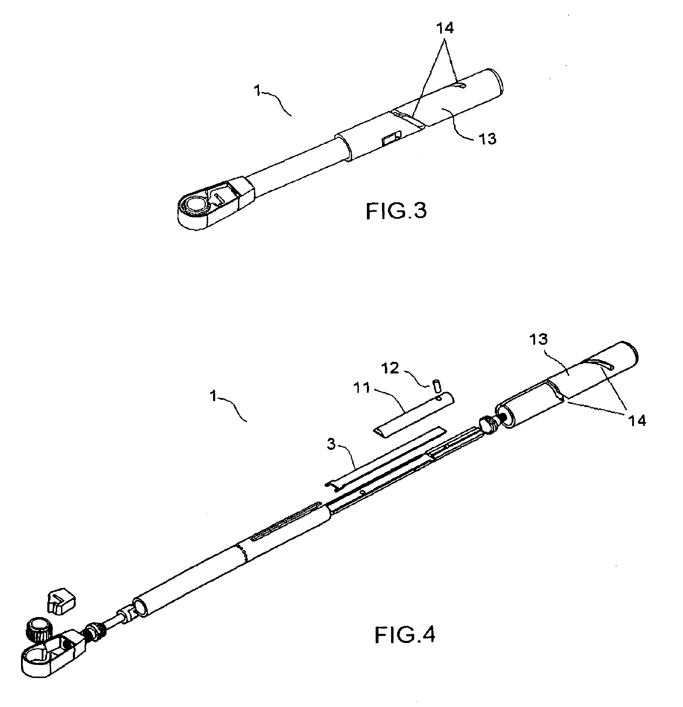

Figures 3 and 4 show a second embodiment of the wrench according to the invention, in which the maximum torque provided by the wrench is variable. In this case the wrench (1) further comprises means for varying the buckling length of the elongated metal part (3) and, as a result, for varying the maximum torque provided by the wrench (1). In this case said means take the form of a sliding part (11) that presses the elongated metal part (3) at a variable point (P), the sliding part (11) being capable of being operated from the outside of the wrench (1).

Preferably, the sliding part (11) is operated from the outside of the wrench (1) by means of a bolt (12). Said bolt (12) is engaged with a moving cover (13), which enables said bolt (12) to be moved. For this purpose, the moving cover (13) preferably presents a helicoidal groove (14) in which the bolt (12) moves, with the result that the rotation of the moving cover (13) causes the bolt (12) to move axially.

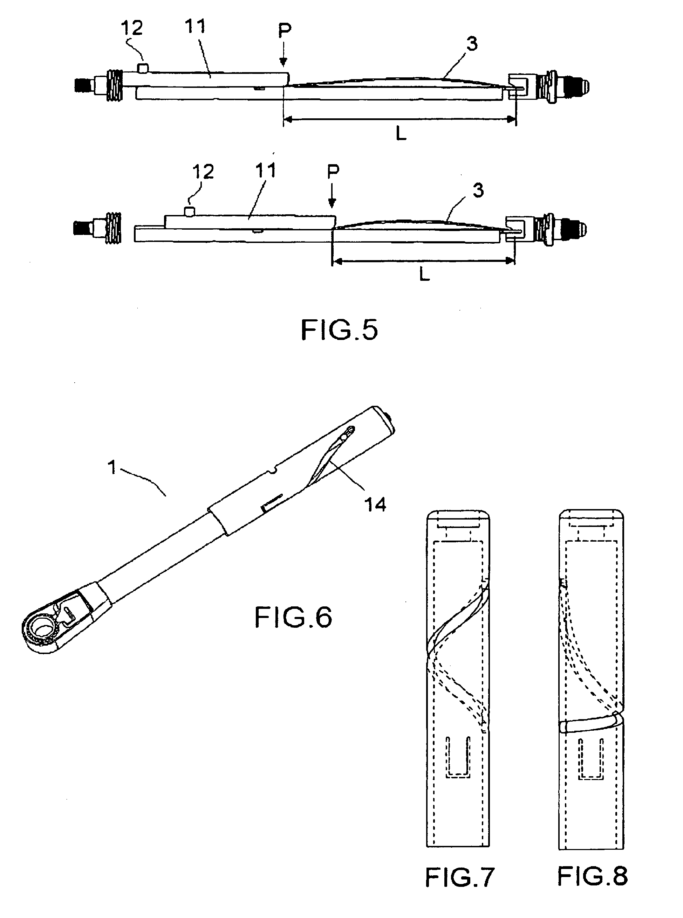

Figure 5 shows two longitudinal cross-sectional views of the wrench (1) of Figures 3 and 4. For sake of clarity not all parts are shown. The two views show how it is possible to select the required torque: depending on the angle at which the moving cover (13) is rotated, the sliding part (11) slides for a certain distance, altering the buckling length (L) and, as a result, the torque applied. In the top figure the sliding part (11) is situated more to the left, with the point (P) being situated as a result at the furthest possible limit on the left. The buckling length (L) of the elongated metal part (3) is therefore very large, with the maximum torque provided by the wrench (1) being relatively low as a result. In the bottom figure, the sliding part (11) and, as a result, the point (P) have moved a certain distance to the right. The buckling length (L) is therefore smaller than in the top figure and the maximum torque provided by the wrench (1) is greater.

In the wrench shown in Figures 3 and 4, the helicoidal groove (14) presents a fixed pitch. In other words, the relationship between the angle of rotation and the torque applied is not linear (equal increases in angle do not correspond with equal increases in torque). The moving cover (13) provided with a fixed-pitch helicoidal groove (14) can be seen in Figure 7.

Alternatively, Figure 6 shows another embodiment of the wrench (1) according to the invention, wherein the maximum torque provided by the wrench is variable, as in Figures 3 and 4, but in which the helicoidal groove (14) presents a variable pitch. In this case, the relationship between the angle of rotation and the torque applied can be linear (equal angles of rotation correspond with equal variations in torque) or as the user requires. In other words, the provision of a variable pitch enables a linear relationship to be established between the angle of rotation of the moving cover (13) and the maximum torque provided by the wrench (1). This makes the adjusting of the maximum torque of the wrench (1) more of an intuitive process for the user. The moving cover (13) provided with a variable-pitch helicoidal groove (14) may be seen in Figure 8.

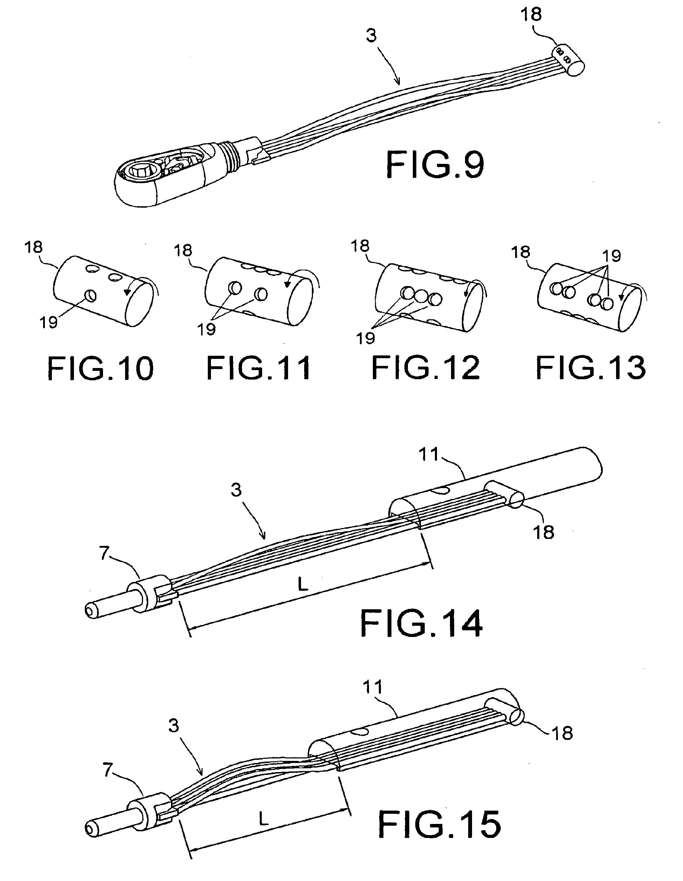

Figure 9 shows an alternative embodiment of the invention. In this case the elongated metal part (3) is in fact a set of several elongated metal parts in the form of rods, in this example five rods in total. The wrench (1) comprises a control part (18) that allows the number of rods capable of being bent to be selected and therefore allows the maximum torque provided by the wrench (1) to be adjusted. In other words, the present embodiment enables the regulation of the momentum of the elongated metal part (3) formed by a set of several elongated metal parts. For this purpose, the control part (18) may rotate and comprises a series of holes (19) designed to allow certain rods to pass through so that said rods cannot buckle. Figures 10 to 13 show the various positions that the control part (18) may adopt to enable the number of buckleable rods to be varied and therefore enable the adjustment of the momentum of the set of rods (and the maximum torque of the wrench as a result). In Figure 10 the control part (18) is situated in a position in which there is only one hole (19) aligned with the rods. As a result, the central rod does not buckle whereas the other four rods (two on either side of the central one) do buckle, the control part (18) acting as a stopper on the latter. In Figure 11 the control part (19) has rotated to a position where there are two holes (19) aligned with the rods, with the result that three rods buckle and two rods do not buckle. In Figure 12 the control part (19) has rotated to a position where there are three holes (19) aligned with the rods, with the result that two rods buckle and three rods do not buckle (arrangement shown in Figure 9). Finally, in Figure 13, the control part (19) has rotated to a position where there are four holes (19) aligned with the rods, with the result that a single rod buckles and the four other rods do not buckle.

Figures 14 and 15 show another embodiment of the invention in which the two preceding concepts are combined (the variation of the point of inertia and the variation of the buckling length). The wrench (1) comprises several elongated metal parts (3) in the form of rods and means for selecting both the number of elongated metal parts (3) capable of being bent and the buckling length of said metal parts (3). Particularly, a control part (18) and a sliding part (11) such as those described in preceding figures are comprised. In Figure 14 the wrench is adjusted in such a way that only one rod may be bent and with a large buckling length (L), whereas in Figure 15 the control part (18) and the sliding part (11) are adjusted in such a way that three rods with a smaller buckling length (L) may be bent.

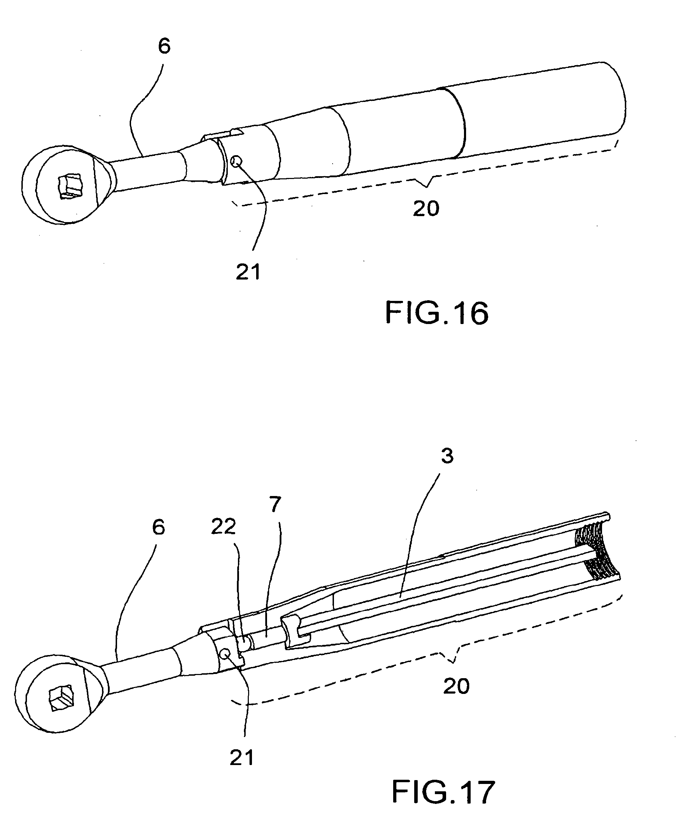

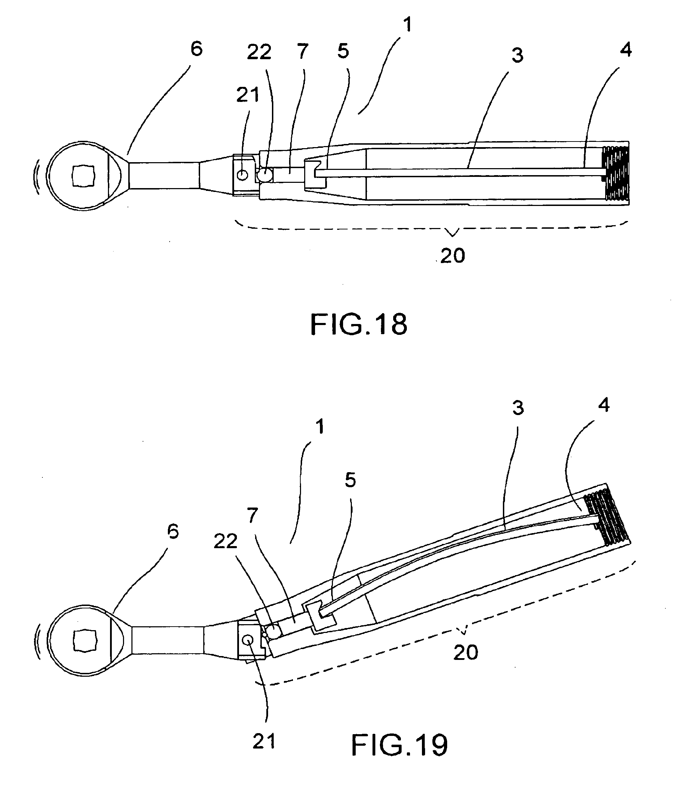

Figures 16 and 17 respectively show a perspective and a cross-sectional perspective of yet another embodiment of the wrench (1) according to the invention. In this case the elongated metal part (3) forms part of a set of parts (20) connected by means of an articulated joint (21) to the torque-applying head (6). Figures 18 and 19 show an elevated cross-sectional view of the wrench (1) in its initial position and in its position of maximum torque respectively.

This embodiment works as follows. The user starts to use the wrench (1) in the position shown in Figure 1, in which the set of parts (20) are aligned with the torque-applying head (6). As the user exerts a increasing force, i.e. as the wrench (1) applies an increasing torque, the set of parts (21) starts to rotate in relation to the torque-applying head (6) and the elongated metal part (3) starts to buckle. The elongated metal part (3) then reaches its maximum buckling point and the set of parts (21) is no longer able to continue rotating in relation to the torque-applying head (6) (a situation shown in Figure 19). At this point the wrench (1) indicates that it has reached its maximum torque.

In this embodiment, therefore, the point at which maximum torque has been reached is indicated by the set of parts (20) rotating in relation to the torque-applying head (6) to the angle of maximum rotation, and not by a "click" or other indication emitted by the torque-applying head, as in the preceding embodiment. In addition, the point where the torque is applied (which is located approximately in the articulated joint (21), in other words right where the pusher member (7) acts) is situated further away from the axis of rotation of the external part (a screw, a nut, etc.) on which torque is to be provided. It is for this reason that, in providing a certain torque, the elongated metal part (3) should not buckle as much in this embodiment as in the preceding embodiment.

In this embodiment the torque is applied on the area of the articulated joint (21), instead of on the catch (17) and the gearwheel (16) of the torque-applying head (6) as was the case in the embodiment shown in the preceding figures. This makes the construction of the wrench (1) easier, as the shape of the parts related to the articulated joint (21) is such that these parts can be relatively easily and cost-effectively manufactured from hard materials (which in turn are able to withstand high torques); the rest of the wrench (1) can be manufactured using materials with a standard hardness.

In the embodiment shown in Figures 16 to 19, the articulated joint (21) is formed by a set of balls (22), although the invention is not limited in this sense and contemplates many alternative or additional embodiments.

## Claims

1. Wrench (1) for providing a maximum torque to an external part, **characterised in that** it comprises a torque-applying head (6) and at least one elongated metal part (3) that pushes the torque-applying head (6), wherein the elongated metal part (3) is formed along a longitudinal direction and is provided with a fixed end (4) and a pusher end (5), wherein the pusher end (5) of the elongated metal part (3) pushes a pusher member (7) that acts on the torque-applying head (6), wherein the pusher end (5) is capable of moving along said longitudinal direction and the elongated metal part (3) is capable of buckling when torque is applied with the wrench, and wherein the elongated metal part (3) reaches a maximum buckling point that determines the maximum torque provided by the wrench (1).

2. Wrench (1), according to claim 1, **characterised in that** the pusher member (7) pushes a catch (17), the elongated metal part (3) being capable of buckling when the catch (17) offers resistance, resulting in the provision of an increasing amount of torque until the elongated metal part (3) reaches a specific buckling point that causes the pusher end (5) to exert sufficient force on the catch (17) so that the catch (17) jumps to a next position, thus resulting in the limiting of the torque provided by the wrench (1).

3. Wrench (1), according to claim 2, **characterised in that** it comprises a fixed cover (2) inside which the elongated metal part (3) is situated and on one end of which the catch (17) is connected.

4. Wrench (1), according to claim 1, **characterised in that** it comprises means for selecting the buckling length of the elongated metal part (3) and, as a result, for varying the maximum torque provided by the wrench (1).

5. Wrench (1), according to claim 4, **characterised in that** the means for selecting the buckling length of the elongated metal part (3) comprise a sliding part (11) that presses the elongated metal part (3) at a variable point (12), the sliding part (11) being capable of being operated on the outside of the wrench (1).

6. Wrench (1), according to claim 5, **characterised in that** the sliding part (11) is capable of being operated on the outside of the wrench (1) by means of a bolt (12).

7. Wrench (1), according to claim 6, **characterised in that** it further comprises a moving cover (13) that is engaged with the bolt (12) and which causes said bolt (12) to move.

8. Wrench (1), according to claim 7, **characterised in that** the moving cover (13) presents a helicoidal groove (14) in which the bolt (12) moves, with the result that a rotation of the moving cover (13) causes the bolt (12) to move axially.

9. Wrench (1), according to claim 8, **characterised in that** the helicoidal groove (14) presents a fixed pitch.

10. Wrench (1), according to claim 8, **characterised in that** the helicoidal groove (14) presents a variable pitch.

11. Wrench (1), according to claim 1, **characterised in that** it comprises one elongated metal part (3).

12. Wrench (1), according to claim 1, **characterised in that** it comprises various elongated metal parts (3) and means for selecting the number of elongated metal parts (3) capable of buckling and, as a result, for altering the maximum torque provided by the wrench (1).

13. Wrench (1), according to claim 1, **characterised in that** the elongated metal part (3) forms part of a set of parts (20) that form an articulated joint with the torque-applying head (6), with the result that when the torque provided by the wrench is increased, said set of parts (20) rotates in relation to the torque-applying head (6) thereby causing the elongated metal part (3) to buckle, until the elongated metal part (3) reaches a specific maximum buckling point, at which point the wrench (1) provides its maximum torque.

## Patentansprüche

1. Schlüssel (1) zum Erzeugen eines maximalen Drehmoments an ein äußeres Teil, **dadurch gekennzeichnet, dass** er einen Drehmoment ausübenden Kopf (6) und mindestens ein längliches Metallteil (3) umfasst, das gegen den Drehmoment ausübenden Kopf (6) schiebt, wobei das längliche Metallteil (3) im einer Längsrichtung gebildet ist und mit einem festen Ende (4) und einem Schieberende (5) versehen ist, wobei das Schieberende (5) des länglichen Metallteils (3) ein Schiebeglied (7) schiebt, das auf den Drehmoment ausübenden Kopf (6) einwirkt, wobei das Schieberende (5) sich in besagter Längsrichtung bewegen kann und das längliche Metallteil (3) in der Lage ist sich zu knicken, wenn durch den Schlüssel ein Drehmoment ausgeübt wird, und wobei das längliche Metallteil (3) eine Sollknickstelle erreicht, die das durch den Schlüssel (1) erzeugende maximale Drehmoment bestimmt.

2. Schlüssel (1), gemäß Anspruch 1, **dadurch gekennzeichnet, dass** das Schiebeglied (7) gegen eine Rastung (17) schiebt, wobei das längliche Metallteil (3) in der Lage ist sich zu knicken, wenn die Rastung (17) Widerstand leistet, was zur einer Erhöhung des Drehmoments führt, bis das längliche Metallteil (3) eine spezifische Knickstelle erreicht, die dazu führt, dass das Schieberende (5) ausreichend Kraft auf die Rastung (17) ausübt, so dass die Rastung (17) in eine nächste Position springt, was zur Begrenzung des durch den Schlüssel (1) erzeugenden Drehmoments führt.

3. Schlüssel (1), gemäß Anspruch 2, **dadurch gekennzeichnet, dass** er eine feste Abdeckung (2) umfasst, in deren Inneres das längliche Metallteil (3) sitzt, und an deren einem Ende die Rastung (17) angeschlossen ist.

4. Schlüssel (1), gemäß Anspruch 1, **dadurch gekennzeichnet, dass** er Mittel für die Auswahl der Knicklänge des länglichen Metallteils (3) und, als Ergebnis davon, für die Variation des durch den Schlüssel (1) erzeugenden maximalen Drehmoments umfasst.

5. Schlüssel (1), gemäß Anspruch 4, **dadurch gekennzeichnet, dass** die Mittel für die Auswahl der Knicklänge des länglichen Metallteils (3) ein Gleitteil (11) umfassen, das an einer variablen Stelle (12) gegen das längliche Metallteil (3) drückt, wobei das Gleitteil (11) von der Außenseite des Schlüssels (1) aus bedienbar ist.

6. Schlüssel (1), gemäß Anspruch 5, **dadurch gekennzeichnet, dass** das Gleitteil (11) von der Außenseite des Schlüssels (1) mit Hilfe eines Bolzens (12) bedienbar ist.

7. Schlüssel (1), gemäß Anspruch 6, **dadurch gekennzeichnet, dass** er ferner eine bewegliche Abdeckung (13) umfasst, die in den Bolzen (12) einrastet und dazu führt, dass sich besagter Bolzen (12) bewegt.

8. Schlüssel (1), gemäß Anspruch 7, **dadurch gekennzeichnet, dass** die bewegliche Abdeckung (13) eine spiralförmige Nut (14) aufweist, in der sich der Bolzen (12) bewegt, mit dem Ergebnis, dass eine Rotation der beweglichen Abdeckung (13) dazu führt, dass der Bolzen (12) sich axial bewegt.

9. Schlüssel (1), gemäß Anspruch 8, **dadurch gekennzeichnet, dass** die spiralförmige Nut (14) ein festen Abstand aufweist.

10. Schlüssel (1), gemäß Anspruch 8, **dadurch gekennzeichnet, dass** die spiralförmige Nut (14) einen variablen Abstand aufweist.

11. Schlüssel (1), gemäß Anspruch 1, **dadurch gekennzeichnet, dass** er ein längliches Metallteil (3) umfasst.

12. Schlüssel (1), gemäß Anspruch 1, **dadurch gekennzeichnet, dass** er mehrere längliche Metallteile (3) und Mittel für die Auswahl der Zahl der länglichen Metallteile (3), die knicken können, und, als Ergebnis, für die Änderung des durch den Schlüssel (1) erzeugenden maximalen Drehmoments umfasst.

13. Schlüssel (1), gemäß Anspruch 1, **dadurch gekennzeichnet, dass** das längliche Metallteil (3) Teil eines Teilesatzes (20) ist, der ein Knickgelenk mit dem Drehmoment ausübenden Kopf (6) bildet, mit dem Ergebnis, dass, wenn das durch den Schlüssel erzeugende Drehmoment erhöht wird, besagter Teilesatz (20) gegenüber dem Drehmoment ausübenden Kopf (6) rotiert, und damit bewirkt, dass sich das längliche Metallteil (3) knickt, bis das längliche Metallteil (3) eine spezifische Sollknickstelle erreicht, an der der Schlüssel (1) sein maximales Drehmoment erzeugt.

## Revendications

1. Clé (1) pour exercer un couple maximal à une pièce externe, **caractérisée en ce qu'**elle comprend une tête de serrage au couple (6) et au moins une pièce métallique allongée (3) qui pousse la tête de serrage au couple (6), la pièce métallique allongée (3) étant disposée le long d'une direction longitudinale et équipée d'une extrémité fixe (4) et d'une extrémité poussoir (5), l'extrémité poussoir (5) de la pièce métallique allongée poussant un élément poussoir (7) qui agit sur la tête de serrage au couple (6), l'extrémité poussoir (5) pouvant se déplacer dans le sens de la longueur et la pièce métallique allongée (3) pouvant se courber quand le couple est appliqué avec la clé, et où la pièce métallique allongée (3) atteint le point de courbure maximal correspondant au couple maximal appliqué par la clé (1).

2. Clé (1), selon la revendication 1, **caractérisée en ce que** l'élément poussoir (7) pousse un cliquet (17), la pièce métallique allongée (3) pouvant se courber quand le cliquet (17) oppose résistance, ce qui conduit à l'application d'un couple croissant jusqu'à ce que la pièce métallique allongée (3) atteigne un point de courbure spécifique qui fait que l'extrémité poussoir (5) exerce une force suffisante sur le cliquet (17) pour que le cliquet (17) saute à la position suivante, la conséquence étant de limiter le couple appliqué par la clé (1).

3. Clé (1), selon la revendication 2, **caractérisée en ce qu'**elle comprend un fourreau fixe (2) à l'intérieur duquel se situent la pièce métallique allongée (3) et l'une des extrémités auquel le cliquet (17) est relié.

4. Clé (1), selon la revendication 1, **caractérisée en ce qu'**elle comprend des moyens de sélection de la longueur de courbure de la pièce métallique allongée (3) ce qui permet de régler le couple maximal fourni par la clé (1).

5. Clé (1), selon la revendication 4, **caractérisée en ce que** les moyens de sélection de la longueur de courbure de la pièce métallique allongée (3) comprennent une pièce coulissante (11) qui appuie sur la pièce métallique allongée (3) au point de réglage (12), la pièce coulissante (11) pouvant être manipulée de l'extérieur de la clé (1).

6. Clé (1), selon la revendication 5, **caractérisée en ce que** la pièce coulissante (11) peut être manipulée de l'extérieur de la clé (1) à l'aide d'un bouton (12).

7. Clé (1), selon la revendication 6, **caractérisée en ce qu'**elle comprend également un fourreau mobile (13) sur lequel le bouton (12) s'emboîte, permettant au bouton (12) de se déplacer.

8. Clé (1) selon la revendication 7, **caractérisée en ce que** le fourreau mobile (13) dispose d'une rainure hélicoïdale (14) dans lequel le bouton (12) se déplace, le résultat étant que la rotation du fourreau mobile (13) permet que le bouton (12) se déplace axialement.

9. Clé (1), selon la revendication 8, **caractérisée en ce que** la rainure hélicoïdale (14) dispose d'un pas fixe.

10. Clé (1), selon la revendication 8, **caractérisée en ce que** la rainure hélicoïdale (14) dispose d'un pas variable.

11. Clé (1), selon la revendication 1, **caractérisée en ce qu'**elle comprend une pièce métallique allongée (3).

12. Clé (1), selon la revendication 1, **caractérisée en ce qu'**elle comprend plusieurs pièces métalliques allongées (3) et des moyens de sélection du nombre des pièces métalliques allongées (3) aptes à la courbure et pouvant donc modifier le couple maximal appliqué par la clé (1).

13. Clé (1), selon la revendication 1, **caractérisée en ce que** la pièce métallique allongée (3) fait partie d'un ensemble de pièces (20) formant un joint articulé avec la tête de serrage au couple (6), le résultat étant que lorsque le couple appliqué par la clé augmente, ledit ensemble de pièces (20) pivote par rapport à la tête de serrage (6) causant une courbure de la pièce métallique allongée (3) jusqu'à ce que la pièce métallique allongée (3) atteigne un point de courbure maximal, point auquel la clé (1) applique le couple maximal.
